(19) Europäisches Patentamt
European Patent Office
Office européen des brevets

(11) **EP 4 108 237 A1**

(12) **EUROPEAN PATENT APPLICATION**
published in accordance with Art. 153(4) EPC

(43) Date of publication:
**28.12.2022 Bulletin 2022/52**

(21) Application number: **21757414.4**

(22) Date of filing: **18.02.2021**

(51) International Patent Classification (IPC):
*A61K 9/70* (2006.01)      *A61K 47/12* (2006.01)
*A61K 47/14* (2017.01)      *A61K 47/32* (2006.01)
*A61K 31/137* (2006.01)

(52) Cooperative Patent Classification (CPC):
**A61K 9/70; A61K 31/137; A61K 47/12;
A61K 47/14; A61K 47/32**

(86) International application number:
**PCT/JP2021/006051**

(87) International publication number:
**WO 2021/166986 (26.08.2021 Gazette 2021/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.02.2020  JP 2020026339**

(71) Applicant: **NITTO DENKO CORPORATION
Ibaraki-shi
Osaka 567-8680 (JP)**

(72) Inventors:
• **FUJIWARA, Kaiji**
**Ibaraki-shi, Osaka 567-8680 (JP)**
• **IMAI, Hiroki**
**Ibaraki-shi, Osaka 567-8680 (JP)**
• **TANAKA, Tomoya**
**Ibaraki-shi, Osaka 567-8680 (JP)**
• **NISHIMURA, Masato**
**Ibaraki-shi, Osaka 567-8680 (JP)**

(74) Representative: **Heubeck, Christian
Weickmann & Weickmann
Patent- und Rechtsanwälte PartmbB
Postfach 860 820
81635 München (DE)**

(54) **PATCH PREPARATION**

(57)    The problem of the present invention is to provide a patch preparation superior in the skin permeability of drugs and having good adhesive property. The present invention relates to a patch preparation containing a drug (excluding tandospirone and a pharmaceutically acceptable salt thereof), levulinic acid, and propylene glycol fatty acid ester in the adhesive layer, which is superior in the practical adhesive property and skin permeability of drugs.

EP 4 108 237 A1

**Description**

[Technical Field]

The present invention relates to a patch preparation containing a drug, levulinic acid, and propylene glycol fatty acid ester.

[Background Art]

Transdermal absorption preparations such as patch preparation and the like have been adopted as preparations not only aiming at treating lesions on the skin surface or in the tissues directly under a skin application site by topical absorption of the drug, but also expected to act systemically by the drug that has permeated through the skin and has further been directly taken into the subcutaneous capillary. Transdermal administration of a drug by such patch preparation or the like has advantages such as being able to suppress the overdosage of the drug because it can avoid the first pass effect in the liver; when the transdermal administration of a drug is to be interrupted, the administration of the drug can be conveniently and safely interrupted by peeling off the patch preparation from the skin surface under application; and the like.

However, the skin to which the patch preparation is applied has a barrier function to prevent intrusion of exogenous substances and the like from the outside. Thus, in many cases, it is difficult to achieve skin permeation of a drug in an amount necessary and sufficient for expressing its efficacy. Given the above, various measures have been conventionally taken to improve the transdermal permeability of drugs.

For example, skin permeability of a drug may be improved by increasing the flat plane area of the patch preparation. However, there were problems such as handleability due to the large flat plane area (difficult to attach to the skin, difficult to replace the patch, etc.), problems of easy occurrence of stuffiness, itching, and the like during application to the skin, and the like (Patent Literature 1). In addition, an attempt has also been studied in order to improve the skin permeability of a drug by containing a transdermal absorption promoter in the patch preparation. Depending on the drug, sufficient skin permeability is difficult to obtain at times, or depending on the kind of transdermal absorption promoter, adverse effects such as deterioration of the adhesive properties (cohesion strength, etc.) of the patch preparation may occur (Patent Literature 2).

[Citation List]

[Patent documents]

[Patent Document 1]
JP-A-10-251145
[Patent Document 2]
JP-A-7-247221

[Summary of Invention]

[Problems to be solved by the Invention]

In view of the above situation, it is an object of the present invention to provide a patch preparation having superior skin permeability of a drug and good adhesive property.

[Means of Solving the Problems]

The present inventors have conducted intensive studies in an attempt to achieve the aforementioned object and found that the practical adhesive property and skin permeability of a drug as a transdermal absorption preparation can be imparted to a patch preparation, as well as skin irritation during detachment can be reduced, by adding a drug (excluding tandospirone and a pharmaceutically acceptable salt thereof), levulinic acid, and propylene glycol fatty acid ester to an adhesive layer of the patch preparation, which resulted in the completion of the present invention.

Accordingly, the present invention provides the following.

[1] A patch preparation comprising a support and an adhesive layer on at least one surface of the support,

wherein the adhesive layer comprises a drug (excluding tandospirone and a pharmaceutically acceptable salt thereof), levulinic acid, and propylene glycol fatty acid ester (hereinafter sometimes to be referred to as "the patch preparation of the present invention").

[2] The patch preparation of the above-mentioned [1], wherein the aforementioned propylene glycol fatty acid ester is an ester of propylene glycol and saturated or unsaturated fatty acid having a carbon number of 8 to 18.

[3] The patch preparation of the above-mentioned [1] or [2], wherein the aforementioned propylene glycol fatty acid ester is propylene glycol monocaprylate or propylene glycol monolaurate.

[4] The patch preparation of any of the above-mentioned [1] to [3], wherein the aforementioned adhesive layer further comprises other fatty acid ester other than the propylene glycol fatty acid ester.

[5] The patch preparation of the above-mentioned [4], wherein the aforementioned other fatty acid ester is at least one kind selected from the group consisting of an ester of alcohol having a linear or branched chain alkyl group or cyclic alkyl group having a carbon number of 1 to 18 and saturated or unsaturated fatty acid having a carbon number of 8 to 18, and an ester of glycerol and saturated or unsaturated fatty acid having a carbon number of 8 to 18.

[6] The patch preparation of any of the above-mentioned [1] to [5], wherein a content of the aforementioned propylene glycol fatty acid ester is 1 to 30 mass % in 100 mass % of the adhesive layer.

[7] The patch preparation of any of the above-mentioned [1] to [6], wherein a content ratio of the levulinic acid and the propylene glycol fatty acid ester is 1:0.1 to 1:20 in mass ratio.

[8] The patch preparation of any of the above-mentioned [1] to [7], wherein an adhesive base constituting the aforementioned adhesive layer is an acrylic polymer.

[9] The patch preparation of any of the above-mentioned [1] to [8], wherein the aforementioned drug is a basic drug having a basic group.

[10] The patch preparation of any of the above-mentioned [1] to [9], wherein the aforementioned drug has a logPow of -1 to 7.

[Effect of the Invention]

According to the present invention, a patch preparation superior in the skin permeability of a drug and having good adhesive properties (cohesion strength, water-resistant adhesiveness, etc.) can be provided.

[Brief Description of Drawings]

[Fig. 1]

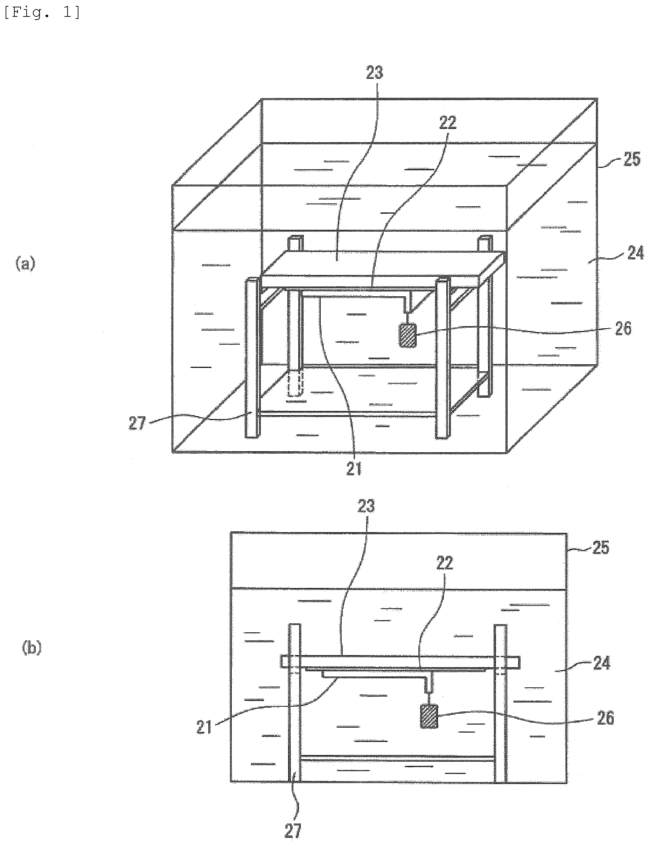

Fig. 1 shows a measurement method for an underwater constant-load peeling test of a patch preparation, wherein (a) is a schematic perspective view thereof, and (b) is a schematic front view thereof. Each dimension and dimensional ratio in the Figures are exaggerated for convenience of explanation and may differ from the actual ratio.

[Description of Embodiments]

Hereinafter, preferred embodiments of the present invention are explained, but the present invention is not limited to these specific embodiments.

Adhesive layer

The patch preparation of the present invention characteristically contains a drug, levulinic acid, and propylene glycol fatty acid ester in the adhesive layer.

(Drug)

The drug in the patch preparation of the present invention is not particularly limited, except that tandospirone and a pharmaceutically acceptable salt thereof are excluded, as long as the drug can be administered to mammals such as human and the like through the skin thereof (that is, transdermally absorbable drug). Examples of such drug include systemic anesthetics, hypnotic sedatives, antiepileptic drugs, antipyretic analgesic antiphlogistic drugs, anti-vertiginous drugs, psychoneurotic drugs, central nervous system drug, antidementia drugs, local anesthetics, skeletal muscle relaxants, autonomic drugs, antiepileptic drugs, antiparkinsonian drugs, antihistamine drugs, cardiotonic drugs, arrhythmia drugs, diuretics, hypotensive drugs, vasoconstrictors, coronary vasodilators, peripheral vasodilators, arteriosclerosis drugs, drugs for circulatory organs, anapnoics, antitussive expectorants, hormone drugs, external drugs for mattery diseases, analgesic/antipruritic/styptic/antiphlogistic drugs, anti-dermoinfectives, hemostats, gout treatment drugs, drugs for diabetes, drugs for anti-malignant tumor, antibiotics, chemotherapeutic drugs, narcotics, smoking cessation drugs, and the like.

The drug may be in the form of a free base or pharmaceutically acceptable salt. The pharmaceutically acceptable salt is not particularly limited, and examples thereof include addition salts of organic acids such as formate, acetate, lactate, adipate, citrate, tartrate, methanesulfonate (also called mesylate), benzenesulfonate (also called besylate), fumarate, maleate, and the like; addition salts of inorganic acids such as hydrochloride, sulfate, nitrate, phosphate, and the like; addition salts of organic bases such as meglumine salt, piperazine salt, tromethamine salt, choline salt, diethylamine salt, tert-butylamine salt, and the like; addition salts of inorganic bases such as sodium salt, calcium salt, potassium salt, magnesium salt, aluminum salt, ammonium salt, and the like; and the like. The drug may be a solvate (e.g., hydrate, ethanol solvate, propylene glycol solvate) or a nonsolvate.

As the drug in the patch preparation of the present invention, a basic drug having a basic group is preferably used. As such drug, a drug having at least one kind of functional group selected from the group consisting of alcoholic hydroxy group, sulfanyl group, phenolic hydroxy group, and amino group (e.g., primary amino group ($-NH_2$), secondary amino group (-NRH), tertiary amino group (-NRR'); wherein R and R' are each independently optionally substituted alkyl group or optionally substituted aryl group.) can be mentioned.

The coefficient of partition of the drug in the patch preparation of the present invention (1-octanol/water), that is, the lower limit value of logPow is preferably -1, more preferably 0, further preferably 1, and the upper limit value of logPow is preferably 7, more preferably 6, further preferably 5. A drug having a logPow of -1 to 7 is superior in the drug dispersibility in the adhesive layer.

As used herein, the logPow is an index showing hydrophilicity or hydrophobicity of a drug. It refers to a value measured for each drug by the method described in "OECD GUIDELINE FOR THE TESTING OF CHEMICALS 107, Adopted by the Council on 27th July 1995, Partition Coefficient (n-octanol/water), Shake Flask Method", wherein the base of the logarithm of logPow is 10. In this embodiment, logPow was calculated using logP calculation software (Scigress manufactured by FUJITSU) . For measurement (calculation) of logPow, the structural formula of the compound is input to the calculation software and logPow is calculated.

The content of the drug in the adhesive layer is not particularly limited as long as the effect of the drug is exhibited, and the side effects are not caused. The lower limit of the content is preferably 0.1 mass %, more preferably 0.5 mass %, further preferably 1 mass %the, in 100 mass % of the adhesive layer. The upper limit of the drug content is preferably 50 mass %, more preferably 40 mass %, further preferably 30 mass %, in 100 mass % of the adhesive layer. When the content of the drug is less than 0.1 mass %, sufficient efficacy may not be achieved, and conversely, when the content of the drug exceeds 50 mass %, the drug has a risk of causing side effects (e.g., skin irritation during application, etc.).

(Levulinic acid)

The levulinic acid used in the present invention is an organic acid classified as keto acid. The levulinic acid may be in the form of a free acid (that is, levulinic acid itself) or a pharmaceutically acceptable salt thereof. Examples of the pharmaceutically acceptable salt include alkali metal salts such as sodium salt and the like, and the like. As the levulinic acid, a commercially available product may be used as it is, or a pharmaceutically acceptable salt thereof prepared from levulinic acid according to a method known per se may be used. As the levulinic acid used in the present invention, levulinic acid as a free acid is preferred.

The content of levulinic acid in the adhesive layer is not particularly limited, and the upper limit of the content of levulinic acid is preferably 30 mass %, more preferably 20 mass %, further preferably 15 mass %, in 100 mass % of the adhesive layer. The lower limit of the content of levulinic acid is preferably 0.1 mass %, more preferably 0.5 mass %, further preferably 1 mass %, in 100 mass % of the adhesive layer. When the content of levulinic acid in the adhesive layer is less than 0.1 mass %, necessary and sufficient skin permeability of the drug is difficult to obtain. On the other hand, when the content of levulinic acid in the adhesive layer exceeds 30 mass %, the shape-retaining property of the adhesive layer may be reduced, oozing out of the adhesive may occur during storage of the patch preparation, or adhesive residue may be left on the skin surface when the patch preparation is peeled off from the skin surface after application thereof.

(Propylene glycol fatty acid ester)

The propyleneglycol fatty acid ester used in the present invention is an ester of propylene glycol and saturated or unsaturated fatty acid having a carbon number of 8 to 18. Examples thereof include propylene glycol mono-fatty acid ester, propylene glycol di-fatty acid ester, and the like. Specific examples of the propylene glycol fatty acid ester include propylene glycol monocaprylate, propylene glycol monocaprate, propylene glycol monolaurate, propylene glycol monostearate, propylene glycol monooleate, propylene glycol dicaprylate, propylene glycol dicaprate, propylene glycol dicaprylate/dicaprate (propylene glycol dicaprylocaprate), propylene glycol dilaurate, propylene glycol distearate, propylene glycol diisostearate, propylene glycol dioleate, and the like. Among them, an ester of propylene glycol and saturated or unsaturated fatty acid having a carbon number of 8 to 14 is preferred, and propylene glycol monocaprylate or propylene glycol monolaurate is more preferred. One kind of these propyleneglycol fatty acid esters may be used, or two or more kinds thereof may be used in combination.

The content of the propyleneglycol fatty acid ester in the adhesive layer is not particularly limited, and the upper limit of the content of the propylene glycol fatty acid ester is preferably 30 mass %, more preferably 25 mass %, further preferably 20 mass %, in 100 mass % of the adhesive layer. The lower limit of the content of the propylene glycol fatty acid ester is preferably 1 mass %, more preferably 2 mass %, further preferably 3 mass %, in 100 mass % of the adhesive layer.

The content ratio of levulinic acid and propylene glycol fatty acid ester (levulinic acid:propylene glycol ester fatty acid) in the adhesive layer is within the range of preferably 1:0.1 to 1:20, more preferably 1:0.2 to 1:15, further preferably 1:0.3 to 1:10, particularly preferably 1:0.5 to 1:10, in mass ratio. When the content of propyleneglycol fatty acid ester in the adhesive layer, or the content ratio of levulinic acid and propyleneglycol fatty acid ester is outside the above-mentioned range, simultaneous achievement of both the good skin permeability of the drug and practical adhesive properties (cohesive strength, water-resistant adhesiveness, etc.) becomes difficult.

(Other fatty acid ester)

In the patch preparation of the present invention, the adhesive layer preferably contains other fatty acid ester other than propylene glycol fatty acid ester. Examples of such other fatty acid ester include fatty acid alkyl esters such as ethyl laurate, isostearyl laurate, isopropyl myristate, isotridecyl myristate, isopropyl palmitate, octyl palmitate, ethyl oleate, and the like (ester of alcohol having a linear or branched chain alkyl group or cyclic alkyl group having a carbon number of 1 to 18 and saturated or unsaturated fatty acid having a carbon number of 8 to 18); glycerol fatty acid esters such as glyceril monocaprylate, glyceril tricaprylate, glyceril tri-2-ethylhexanoate, glyceril tricaprate, glyceril trilaurate, glyceril triisostearate, glyceril trioleate, and the like (ester of glycerol and saturated or unsaturated fatty acid having a carbon number of 8 to 18), and the like. One kind of these other fatty acid esters may be used, or two or more kinds thereof may be used in combination.

The content of other fatty acid ester in the adhesive layer is not particularly limited, and the upper limit of the content of other fatty acid ester is preferably 85 mass %, more preferably 80 mass %, further preferably 75 mass %, in 100 mass % of the adhesive layer. The lower limit of the content of other fatty acid ester is preferably 5 mass %, more preferably 7.5 mass %, further preferably 10 mass %, in 100 mass % of the adhesive layer.

(Adhesive base)

For the expression of good skin adhesiveness, the adhesive layer in the patch preparation of the present invention is preferably an adhesive layer containing an anhydrous adhesive base. Such anhydrous adhesive layer is not limited to one completely free of water, and those containing a little amount of water (e.g., less than 1 mass % in the adhesive layer) derived from moisture in the air, skin, and the like are included.

The adhesive base means a polymer constituting a matrix of the adhesive layer. The adhesive base is blended with the below-mentioned tackifier as necessary to constitute an adhesive. Only one kind of the adhesive base may be used, or two or more kinds thereof may be used in combination. Examples of the anhydrous adhesive base include acrylic polymers such as (meth)acrylate-based polymer and the like; rubber-based polymers such as styrene-isoprene-styrene block copolymer, styrene-butadiene-styrene block copolymer, polyisoprene, polyisobutylene, polybutadiene, and the like; silicone-based polymers such as silicone rubber, dimethylsiloxane base, diphenylsiloxane base, and the like, and the like. Among such anhydrous adhesive bases, an acrylic polymer is preferably contained because drug release and adhesive force can be adjusted easily, irritation to the skin is small, and the like. Such acrylic polymer is not particularly limited, and, for example, an acrylic polymer obtained from a monomer mixture containing alkyl (meth)acrylate monomers is preferably used. In the present specification, the "(meth)acrylic" means both "acrylic" and "methacrylic".

The alkyl (meth)acrylate monomer (hereinafter sometimes to be referred to as "the first monomer") is not particularly limited, and an alkyl (meth)acrylate monomer having an alkyl group having a carbon number of not less than 4 is preferred from the aspect of adhesiveness. Examples of such alkyl (meth)acrylate monomer include an alkyl (meth)acrylate monomer whose alkyl group is a linear alkyl group or branched chain alkyl group having a carbon number of 4 to 13, such as n-butyl group, isobutyl group, sec-butyl group, tert-butyl group, n-pentyl group, isopentyl group, neopentyl group, hexyl group, heptyl group, octyl group, 2-ethylhexyl group, nonyl group, decyl group, undecyl group, dodecyl group, tridecyl group, or the like. Among them, butyl (meth)acrylate and 2-ethylhexyl (meth)acrylate are preferred, and 2-ethylhexyl acrylate is particularly preferred. One kind of these alkyl (meth)acrylate monomers can be used, or two or more kinds thereof can be used in combination.

As the aforementioned acrylic polymer, an acrylic polymer obtained by copolymerizing a monomer mixture containing N-vinylcyclic amides (hereinafter sometimes to be referred to as "the second monomer") in addition to alkyl (meth)acrylate monomer is preferred. Examples of such N-vinylcyclic amide include N-vinyl-2-pyrrolidone, N-vinyl-2-piperidone, N-vinyl-3-morpholinone, N-vinyl-2-caprolactam, N-vinyl-1,3-oxazin-2-one, N-vinyl-3,5-morpholinedione, and the like. Among them, N-vinyl-2-pyrrolidone is particularly preferred. One kind of these N-vinylcyclic amides may be used, or two or more kinds thereof may be used in combination.

When N-vinylcyclic amide is contained as a monomer component constituting an acrylic polymer, the content thereof in the acrylic polymer is preferably not more than 75 mass %, more preferably 1 to 75 mass %, further preferably 5 to 75 mass %, particularly preferably 10 to 70 mass %, most preferably 15 to 65 mass %. By copolymerizing N-vinylcyclic amides, the adhesive force and cohesion strength of the patch preparation can be adjusted, and the solubility of a drug in the adhesive layer and the release of a drug from the adhesive layer can be adjusted. When the content of N-vinylcyclic amide in the acrylic polymer exceeds 75 mass %, the tackiness or adhesive force of the obtained patch preparation may decrease in some cases.

An acrylic polymer obtained by copolymerizing a monomer mixture further containing monomers having other functional group (hereinafter sometimes to be referred to as "the third monomer") as a monomer component constituting the acrylic polymer may be used. Examples of such other functional group include carboxyl group, hydroxy group, vinyl group, and the like. Among them, carboxyl group and hydroxy group are preferred. Specific examples of the monomer having other functional group include (meth)acrylic acid, itaconic acid, (anhydrous) maleic acid, mesaconic acid, citraconic acid, glutaconic acid, hydroxyethyl (meth)acrylate, hydroxypropyl (meth)acrylate, N-hydroxyalkyl(meth)acryl amide, and the like. The N-hydroxyalkyl(meth)acryl amide is preferably N-hydroxyalkyl(C1-4)(meth)acryl amide in which alkyl group has a carbon number of 1 to 4, more preferably N-hydroxyalkyl(C2-4)acrylamide in which alkyl group has a carbon number of 2 to 4. The alkyl group in the hydroxyalkyl group may be a linear or branched chain. Specific examples of the N-hydroxyalkyl(meth)acrylamide include N-(2-hydroxyethyl)acrylamide, N-(2-hydroxyethyl)methacrylamide, N-(2-hydroxypropyl)acrylamide, N-(2-hydroxypropyl)methacrylamide, N-(1-hydroxypropyl)acrylamide, N-(1-hydroxypropyl)methacrylamide, N-(3-hydroxypropyl)acrylamide, N-(3-hydroxypropyl)methacrylamide, N-(2-hydroxybutyl)acrylamide, N-(2-hydroxybutyl)methacrylamide, N-(3-hydroxybutyl)acrylamide, N-(3-hydroxybutyl)methacrylamide, N-(4-hydroxybutyl)acrylamide, N-(4-hydroxybutyl)methacrylamide, and the like. As the monomer having other functional group, acrylic acid, methacrylic acid, hydroxyethyl acrylate, N-(2-hydroxyethyl)acrylamide, or N-(2-hydroxyethyl)methacrylamide is preferred, acrylic acid, hydroxyethyl acrylate, or N-(2-hydroxyethyl)acrylamide is more preferred. One kind of these monomers having other functional group may be used, or two or more kinds thereof may be used in combination.

When the acrylic polymer is a copolymer of alkyl (meth)acrylate (the first monomer), N-vinylcyclic amide (the second monomer), and a monomer having other functional group (the third monomer), the copolymerization ratio thereof (the first monomer/the second monomer/the third monomer) is preferably 20 to 90 mass %/5 to 75 mass %/1 to 15 mass %, more preferably 25 to 85 mass %/10 to 70 mass %/1 to 10 mass %, particularly preferably 30 to 80 mass %/15 to 65 mass %/1 to 10 mass %.

The acrylic polymer can be obtained, for example, by a known polymerization method such as solution polymerization method, emulsion polymerization method, suspension polymerization method, or the like. It can also be obtained by radical polymerization using a radical polymerization initiator such as peroxide compound (e.g., benzoyl peroxide, etc.), azo compound (e.g., azobisisobutyronitrile, etc.), or the like.

When the adhesive base is insufficient in the adhesiveness at ordinary temperature, a tackifier may be further added to impart adhesiveness at ordinary temperature to the adhesive layer. When a rubber-based polymer is used as the adhesive base, as well as when an acrylic polymer is used, a tackifier may be further contained to enhance the adhesiveness of the adhesive layer. As the tackifier, those known in the field of patch preparations can be appropriately selected and used. Specific examples of the tackifier include petroleumbased resin (e.g., aromatic petroleum resin, aliphatic petroleum resin, etc.), terpene-based resin, rosin-based resin, coumarone indene resin, styrene-based resin (e.g., styrene resin, poly($\alpha$-methylstyrene), etc.), hydrogenated petroleum resin (e.g., alicyclic saturated hydrocarbon resin, etc.), and the like. Among them, an alicyclic saturated hydrocarbon resin is preferred because the preservation stability of drug is improved. When a tackifier is used, the content thereof in the adhesive layer is generally not less than 30 parts by mass and less than 100 parts by mass, preferably not less than 50 parts by mass and less than 100 parts by mass, with respect to 100 parts by mass of the adhesive base.

(Crosslinking agent)

In the patch preparation of the present invention, a crosslinking treatment can be performed as necessary to impart appropriate cohesion strength to the adhesive layer. As the crosslinking treatment, a physical crosslinking treatment (e.g., crosslinking treatment by $\gamma$ ray irradiation, electron beam irradiation, or the like), a chemical crosslinking treatment (e.g., crosslinking treatment with a crosslinking agent such as organic peroxide, isocyanate compound, organometallic salt, metal alcholate, metal chelate compound, epoxy-based compound, primary amino group-containing compound, or the like), and the like can be mentioned. Specific examples of the aforementioned chemical crosslinking treatment include a crosslinking treatment with a crosslinking agent such as organic peroxide (e.g., benzoyl peroxide or the like), an isocyanate compound (e.g., tolylene diisocyanate, hexamethylene diisocyanate, or the like), an epoxy compound (e.g., glycerol triglycidyl ether, triglycidyl isocyanurate, or the like), a metal chelate compound (e.g., aluminum tris(acetylacetonate), aluminum ethylacetoacetate diisopropylate, or the like), or the like. Among these crosslinking agents, an isocyanate compound, metal alcholate, or a metal chelate compound is preferably used from the aspects of crosslinking reactivity and easy handling. By such crosslinking treatment, the balance between flexibility and adhesive properties such as cohesion strength and the like of the adhesive layer can be preferably adjusted, which in turn makes it possible to suppress adhesive residue during detachment.

The amount of the crosslinking agent to be added can be appropriately adjusted depending on the kind of the adhesive base or crosslinking agent. It is preferably 0.03 to 2.0 parts by mass, more preferably 0.05 to 1.5 parts by mass, further preferably 0.07 to 1.0 parts by mass, with respect to 100 parts by mass of the adhesive base to be crosslinked (in the case of acrylic polymer).

(Other component (additive))

The adhesive layer of the patch preparation of the present invention may contain, where necessary, various additives such as flavor, colorant, plasticizer, softening agent, filler, stabilizer, antioxidant, antibacterial agent, antimicrobial agent, and the like as appropriate as long as the effect of the present invention is not impaired. As the additives, those known in the field of patch preparations can be appropriately selected and used.

In the patch preparation of the present invention, the upper limit of the thickness of the adhesive layer is preferably 500 $\mu$m, more preferably 400 $\mu$m, further preferably 300 $\mu$m. The lower limit of the thickness of the adhesive layer is preferably 10 $\mu$m, more preferably 20 $\mu$m, further preferably 30 $\mu$m. When the thickness of the adhesive layer is less than 10 $\mu$m, sufficient adhesive property is difficult to obtain, whereas when the thickness of the adhesive layer exceeds 500 $\mu$m, oozing out of the adhesive may occur during storage of the patch preparation.

Support

The support constituting the patch preparation of the present invention is not particularly limited as long as an adhesive layer can be formed and retained on one surface thereof. Examples of the material of the support include polyester (e.g., polyethylene terephthalate (PET), etc.), polyamide (e.g., nylon, etc.), polyethylene, polypropylene, polyvinyl chloride, polyvinylidene chloride (trade name: Saran, etc.), ionomer resin (trade name: Surlyn, etc.), polytetrafluoroethylene, ethylene-ethyl acrylate copolymer, ethylene-vinyl alcohol copolymer (trade name: EVAL, etc.), various metals, and the like. The support may be a single layer such as various plastic films and various metal foils made of the above-mentioned materials, and the like, or may be a laminate thereof. The surface of the support can also be subjected to a treatment with an undercoat layer, a corona discharge treatment, a plasma irradiation treatment, a primer treatment, or the like so as to improve the anchoring property to the adhesive layer formed on the support.

Furthermore, for the purpose of improving the anchoring property of the support to the adhesive layer, a laminate in which a porous sheet is laminated on the adhesive layer-forming side of the support can be used. Specific examples of such porous sheet include aggregates of fibers such as paper, knitted fabric, woven fabric, non-woven fabric (e.g., polyester non-woven fabric, polyethylene terephthalate non-woven fabric, etc.), and the like, sheets such as a single layer of the aforementioned plastic film and the like (single layer film), a laminate film obtained by laminating one or two or more kinds of theses films, and the like, each subjected to a mechanical perforation treatment, and the like. Among these, paper, woven fabric, non-woven fabric (e.g., polyester non-woven fabric, polyethylene terephthalate non-woven fabric, etc.) are preferred.

The thickness of the support is preferably 10 to 200 $\mu$m, more preferably 10 to 100 $\mu$m, from the aspects of improvement of the anchoring property, flexibility and patch application operability of the patch preparation, and the like. When woven fabric or non-woven fabric is used as the porous sheet, the fabric weight of these is preferably 5 to 30 g/m$^2$, more preferably 6 to 15 g/m$^2$.

Release liner

In the patch preparation of the present invention, the surface on the side opposite to the support side of the adhesive layer (adhesion surface to the skin) is preferably laminated with a release liner until actual use of the preparation. The release liner is not particularly limited as long as it has good release property when in use. Specifically, plastic films such as polyester, polyvinyl chloride, polyvinylidene chloride, polyethylene terephthalate, and the like, papers such as high-quality paper, glassine, and the like, and laminate films of polyolefin and paper such as high-quality paper, glassine, and the like (the side to be in contact with the adhesive layer being paper), and the like, each subjected to a release treatment by applying a silicone resin, a fluorine resin, or the like onto the surface to be in contact with the adhesive layer, can be mentioned.

The thickness (total thickness) of the release liner is not particularly limited. It is preferably 10 to 200 $\mu$m, more preferably 25 to 100 $\mu$m.

The size of the patch preparation of the present invention is not particularly limited. It is generally 1 to 300 $cm^2$, preferably 2 to 200 $cm^2$, more preferably 3 to 100 $cm^2$. The patch preparation of the present invention is generally changed to a new one at a frequency of 3 times/day to once/2 weeks, preferably twice/day to once/week, more preferably once/day to once/week, further preferably once/day to once/4 days.

Production method of the patch preparation of the present invention

While the production method of the patch preparation of the present invention is not particularly limited, it can be produced by, for example, the following production method.

An adhesive base containing an acrylic polymer, a drug, levulinic acid, and propylene glycol fatty acid ester are added, together with other additives as necessary, to a suitable solvent and the mixture is sufficiently mixed until it becomes homogeneous. Examples of the solvent include ethyl acetate, toluene, hexane, 2-propanol, methanol, ethanol, water, and the like. When a crosslinking agent is added, it is added to the mixture and the mixture is sufficiently mixed. Where necessary, a solvent may be added along with a crosslinking agent and they are mixed.

Then, the obtained mixture is applied to one surface of the support or a release treating surface of the release liner, and dried to form an adhesive layer. The aforementioned application can be performed by, for example, casting, printing and other techniques known per se to those of ordinary skill in the art. Thereafter, a release liner or support is adhered to the adhesive layer to form a laminate. When a crosslinking treatment is performed, the release liner or support is adhered to the adhesive layer, and they are left standing generally at room temperature to 120°C, preferably room temperature to 100°C, for 8 to 48 hr to promote the crosslinking reaction, whereby a crosslinked adhesive layer is formed.

[Example]

The effect of the present invention is explained more specifically in the following by referring to Examples, Comparative Examples, and Experimental Examples; however, the present invention is not limited by them in any manner. In the following, "parts" and "%" respectively mean "parts by mass" and "mass %". In addition, room temperature shows a temperature of from 15°C to 30°C, unless particularly noted.

[Preparation of acrylic polymer (A)]

Under an inert gas atmosphere, 2-ethylhexyl acrylate (55 parts by mass), N-vinyl-2-pyrrolidone (40 parts by mass), N-(2-hydroxyethyl)acrylamide (5 parts by mass), and 2,2'-azobisisobutyronitrile (0.2 parts by mass) were subjected to solution polymerization in ethyl acetate (60°C) to prepare an acrylic polymer (A) solution.

[Production of patch preparation]

(Examples 1 to 2, Comparative Examples 1 to 5)

The acrylic polymer (A) solution was applied to a release-treated surface of a polyester release liner (thickness: 75 $\mu$m) such that the thickness after drying was 100 $\mu$m, and dried at 100°C for 3 min to form an adhesive layer. The formed adhesive layer was punched into 3 mm$\varphi$, and impregnated with the mixed solution (5 $\mu$L) shown in Table 1 to give a patch preparation. The content (mass %) of each component of the patch preparation is as shown in Table 2.

[Table 1]

|  | Example 1 | Example 2 | Comparative Example 1 | Comparative Example 2 | Comparative Example 3 | Comparative Example 4 | Comparative Example 5 |
|---|---|---|---|---|---|---|---|
| drug | fingolimod 1.87 parts | fingolimod 1.87 parts | fingolimod 7.89 parts | fingolimod 7.89 parts | fingolimod 1.87 parts | fingolimod 8.65 parts | fingolimod 2.06 parts |
| additive 1 | levulinic acid 4.67 parts | levulinic acid 4.67 parts | lactic acid 4.39 parts | lactic acid 4.39 parts | levulinic acid 4.67 parts | lactic acid 4.81 parts | levulinic acid 5.15 parts |
| additive 2 | PGMC 9.35 parts | PGML 9.35 parts | PGMC 8.77 parts | PGML 8.77 parts | DIPA 9.35 parts |  |  |
| additive 3 | IPM 84.11 parts | IPM 84.11 parts | IPM 78.95 parts | IPM 78.95 parts | IPM 84.11 parts | IPM 86.54 parts | IPM 92.78 parts |

PGMC: propylene glycol monocaprylate
PGML: propylene glycol monolaurate
IPM: isopropyl myristate
DIPA: diisopropyl adipate

[Table 2]

|  | Example 1 | Example 2 | Comparative Example 1 | Comparative Example 2 | Comparative Example 3 | Comparative Example 4 | Comparative Example 5 |
|---|---|---|---|---|---|---|---|
| drug | fingolimod 1.6% | fingolimod 1.6% | fingolimod 6.8% | fingolimod 6.8% | fingolimod 1.6% | fingolimod 7.47% | fingolimod 1.78% |
| additive 1 | levulinic acid 4.0% | levulinic acid 4.0% | lactic acid 3.8% | lactic acid 3.8% | levulinic acid 4.0% | lactic acid 4.16% | levulinic acid 4.45% |
| additive 2 | PGMC 8.1% | PGML 8.1% | PGMC 7.6% | PGML 7.6% | DIPA 8.1% |  |  |
| additive 3 | IPM 72.7% | IPM 72.7% | IPM 68.2% | IPM 68.2% | IPM 72.7% | IPM 74.77% | IPM 80.17% |
| adhesive | acrylic polymer (A) 13.6% | acrylic polymer (A) 13.6% | acrylic polymer (A) 13.6% | acrylic polymer (A) 13.6% | acrylic polymer (A) 13.6% | acrylic polymer (A) 13.6% | acrylic polymer (A) 13.6% |

[Skin permeability test 1]

The release liner was peeled off from the above-mentioned 3 mmφ patch preparation and the patch preparation was adhered onto the stratum corneum layer surface of the skin (intact skin) removed from the abdomen or back of hairless mice. The removed skin to which the patch preparation was adhered and fixed was attached to a permeability test device, the receptor solution after 48 hr was sampled, and the skin permeation amount of the drug was calculated by ultra-performance liquid chromatography (UPLC). The receptor solution used was obtained by dissolving 40 g of bovine serum-derived albumin in 1 L of phosphate buffered saline, and the temperature was set to 32°C. The results of the skin permeation amount of the drug in the skin permeability test 1 are shown in Table 3.

[Table 3]

|  | skin permeation amount ($\mu$g/cm$^2$/48h) |
|---|---|
| Example 1 | 53.5 |
| Example 2 | 54.4 |
| Comparative Example 1 | 5.5 |
| Comparative Example 2 | 9.7 |
| Comparative Example 3 | 18.5 |
| Comparative Example 4 | 2.7 |
| Comparative Example 5 | 1.7 |

From the results of Table 3, it was found that the patch preparations of Examples 1 and 2 show remarkably high skin permeability of the drug compared with the patch preparations of Comparative Examples 1 to 5.

[Preparation of acrylic polymer (B)]

Under an inert gas atmosphere, 2-ethylhexyl acrylate (72 parts by mass), N-vinyl-2-pyrrolidone (25 parts by mass), acrylic acid (3 parts by mass), and 2,2'-azobisisobutyronitrile (0.2 parts by mass) were subjected to solution polymerization in ethyl acetate (60°C) to give an acrylic polymer (B) solution.

[Production of patch preparation]

(Examples 3 to 14, Comparative Examples 6 to 29)

A drug, isopropyl myristate (IPM), propylene glycol fatty acid ester (PG fatty acid ester), organic acid, and acrylic polymer (B) solution were added into a container, and the mixture was stirred until it became homogeneous. Aluminum ethylacetoacetate diisopropylate (ALCH) was added, and the viscosity was adjusted with ethyl acetate. The obtained solution was applied to a release-treated surface of a polyester release liner (thickness: 75 $\mu$m) such that the thickness after drying was 100 $\mu$m, and dried at 100°C for 3 min to form an adhesive layer.
The formed adhesive layer was adhered to the non-woven fabric side of the support which is a laminate film of a 2 $\mu$m-thick polyester film and polyester non-woven fabric (fabric weight: 12 g/m$^2$) to give a patch preparation. The content (mass %) of each component of the patch preparation is as shown in Tables 4 and 5.

[Table 4]

| | drug | | organic acid | | PG fatty acid ester | | IPM content (%) | acrylic polymer (B) content (%) | ALCH content (%) |
|---|---|---|---|---|---|---|---|---|---|
| | kind | content (%) | kind | content (%) | kind | content (%) | | | |
| Example 3 | agomelatine | 3.00 | levulinic acid | 1.43 | PGML | 1.43 | 53.94 | 40.00 | 0.20 |
| Example 4 | agomelatine | 3.00 | levulinic acid | 1.43 | PGML | 4.29 | 51.08 | 40.00 | 0.20 |
| Example 5 | agomelatine | 3.00 | levulinic acid | 1.43 | PGML | 7.15 | 48.22 | 40.00 | 0.20 |
| Example 6 | agomelatine | 3.00 | levulinic acid | 1.43 | PGML | 10.01 | 45.36 | 40.00 | 0.20 |
| Comparative Example 6 | agomelatine | 3.00 | levulinic acid | 1.43 | - | - | 55.69 | 40.00 | 0.20 |
| Comparative Example 7 | agomelatine | 3.00 | - | - | PGML | 1.43 | 55.37 | 40.00 | 0.20 |
| Comparative Example 8 | agomelatine | 3.00 | lactic acid | 1.11 | - | - | 55.69 | 40.00 | 0.20 |
| Comparative Example 9 | agomelatine | 3.00 | lactic acid | 1.11 | PGML | 1.11 | 54.58 | 40.00 | 0.20 |
| Comparative Example 10 | agomelatine | 3.00 | lactic acid | 1.11 | PGML | 3.33 | 52.36 | 40.00 | 0.20 |
| Comparative Example 11 | agomelatine | 3.00 | lactic acid | 1.11 | PGML | 5.55 | 50.14 | 40.00 | 0.20 |
| Comparative Example 12 | agomelatine | 3.00 | lactic acid | 1.11 | PGML | 7.77 | 47.92 | 40.00 | 0.20 |
| Example 7 | agomelatine | 3.00 | levulinic acid | 1.43 | PGMC | 1.43 | 53.94 | 40.00 | 0.20 |
| Example 8 | agomelatine | 3.00 | levulinic acid | 1.43 | PGMC | 4.29 | 51.08 | 40.00 | 0.20 |
| Comparative Example 13 | agomelatine | 3.00 | levulinic acid | 1.43 | - | - | 55.37 | 40.00 | 0.20 |

(continued)

| | drug | | organic acid | | PG fatty acid ester | | IPM content (%) | acrylic polymer (B) content (%) | ALCH content (%) |
|---|---|---|---|---|---|---|---|---|---|
| | kind | content (%) | kind | content (%) | kind | content (%) | | | |
| Comparative Example 14 | agomelatine | 3.00 | - | - | PGMC | 1.43 | 55.37 | 40.00 | 0.20 |
| Comparative Example 15 | agomelatine | 3.00 | lactic acid | 1.11 | - | - | 55.69 | 40.00 | 0.20 |
| Comparative Example 16 | agomelatine | 3.00 | lactic acid | 1.11 | PGMC | 1.11 | 54.58 | 40.00 | 0.20 |
| Comparative Example 17 | agomelatine | 3.00 | lactic acid | 1.11 | PGMC | 3.33 | 52.36 | 40.00 | 0.20 |

PGML: propylene glycol monolaurate
PGMC: propylene glycol monocaprylate

[Table 5]

| | drug | | organic acid | | PG fatty acid ester | | IPM content (%) | acrylic polymer (B) content (%) | ALCH content (%) |
|---|---|---|---|---|---|---|---|---|---|
| | kind | content (%) | kind | content (%) | kind | content (%) | | | |
| Example 9 | lidocaine | 3.00 | levulinic acid | 1.49 | PGML | 1.49 | 53.82 | 40.00 | 0.20 |
| Example 10 | lidocaine | 3.00 | levulinic acid | 1.49 | PGML | 4.47 | 50.84 | 40.00 | 0.20 |
| Example 11 | lidocaine | 3.00 | levulinic acid | 1.49 | PGML | 7.45 | 47.86 | 40.00 | 0.20 |
| Comparative Example 18 | lidocaine | 3.00 | levulinic acid | 1.49 | - | - | 55.31 | 40.00 | 0.20 |
| Comparative Example 19 | lidocaine | 3.00 | - | - | PGML | 1.49 | 55.31 | 40.00 | 0.20 |
| Comparative Example 20 | lidocaine | 3.00 | lactic acid | 1.15 | - | - | 55.65 | 40.00 | 0.20 |
| Comparative Example 21 | lidocaine | 3.00 | lactic acid | 1.15 | PGML | 1.15 | 54.50 | 40.00 | 0.20 |
| Comparative Example 22 | lidocaine | 3.00 | lactic acid | 1.15 | PGML | 3.45 | 52.20 | 40.00 | 0.20 |
| Comparative Example 23 | lidocaine | 3.00 | lactic acid | 1.15 | PGML | 5.75 | 49.90 | 40.00 | 0.20 |
| Example 12 | lidocaine | 3.00 | levulinic acid | 1.49 | PGMC | 1.49 | 53.82 | 40.00 | 0.20 |
| Example 13 | lidocaine | 3.00 | levulinic acid | 1.49 | PGMC | 4.47 | 50.84 | 40.00 | 0.20 |
| Example 14 | lidocaine | 3.00 | levulinic acid | 1.49 | PGMC | 7.45 | 47.86 | 40.00 | 0.20 |
| Comparative Example 24 | lidocaine | 3.00 | levulinic acid | 1.49 | - | - | 55.31 | 40.00 | 0.20 |
| Comparative Example 25 | lidocaine | 3.00 | - | - | PGMC | 1.49 | 55.31 | 40.00 | 0.20 |
| Comparative Example 26 | lidocaine | 3.00 | lactic acid | 1.15 | - | - | 55.65 | 40.00 | 0.20 |
| Comparative Example 27 | lidocaine | 3.00 | lactic acid | 1.15 | PGMC | 1.15 | 54.50 | 40.00 | 0.20 |
| Comparative Example 28 | lidocaine | 3.00 | lactic acid | 1.15 | PGMC | 3.45 | 52.20 | 40.00 | 0.20 |
| Comparative Example 29 | lidocaine | 3.00 | lactic acid | 1.15 | PGMC | 5.75 | 49.90 | 40.00 | 0.20 |

[Skin permeability test 2]

The patch preparations of Examples 3 to 14 and Comparative Examples 6 to 29 were punched into 6 mmφ, and the release liner was peeled off. The adhesive layer surface of the above-mentioned patch preparation was adhered onto the stratum corneum layer surface of the skin (intact skin) removed from the abdomen or back of hairless mice. The removed skin to which the patch preparation was adhered and fixed was attached to a permeability test device, the receptor solution after 24 hr was sampled, and the skin permeation amount of the drug was calculated by ultra-performance liquid chromatography (UPLC). The receptor solution used was phosphate buffered saline (9.6 g/L), and the temperature was set to 32°C.

[Cohesion strength test]

The release liner of the patch preparations of Examples 3 to 14 and Comparative Examples 6 to 29 was peeled off, and the exposed surface of the adhesive layer was pressed by a finger for about one second. The state of the adhesive layer and the surface of the finger when the finger was released from the adhesive layer were visually evaluated according to the following indexes.

    ○: no threading and no adhesive remains on the finger
    ×: threading occurs and adhesive remains on the finger

[Underwater constant-load peeling test]

The underwater constant-load peeling test is explained with reference to Fig. 1.
A test plate was prepared by laminating a collagen film 22 (width 25 mm, length 100 mm, thickness 38 μm) on a bakelite board 23 (width 30 mm, length 130 mm, thickness 2 mm) via a double-sided tape (not shown). Then, the patch preparation 21 was cut into a rectangular shape having width 10 mm and length 40 mm, the release paper was peeled off to expose the adhesive layer surface, and then the adhesive layer surface was pressure-bonded to a collagen film 22 of the test plate by one reciprocation with a 2 kg rubber roller to give a test sample. After lapse of 10 min in this state at room temperature, a supporting tool 27 having the shape shown in Fig. 1 was installed in a water tank 25 containing water 24 at 40°C. Furthermore, the test sample was placed such that the two separated ends of the bakelite board 23 were placed on the supporting tool 27 in the state shown in Fig. 1. A 10 g weight 26 was hung on one end of the patch preparation 21, and the distance and time when the patch preparation 21 was peeled off were measured. The same test was repeated three times, the peel rate was calculated from the following formula (1), the average value thereof was taken, and this was calculated as the value of the underwater constant-load peeling test. A smaller value thereof shows that it is more difficult to peel off the patch preparation in water.
[Numerical formula 1]

```
Peel rate (mm/min)=distance peeled (mm)/peeling time (min)  (1)
```

The results of the skin permeability test 2, the cohesion strength test, and the underwater constant-load peeling test are shown in Tables 6 and 7.

[Table 6]

| | skin permeability test permeation amount ($\mu$g/cm$^2$/24hr) | cohesion strength test | underwater constant-load peeling test peel rate (mm/min) |
|---|---|---|---|
| Example 3 | 51.3 | ○ | 78.9 |
| Example 4 | 51.4 | ○ | 111.1 |
| Example 5 | 62.9 | ○ | 120.0 |
| Example 6 | 58.5 | ○ | 130.4 |
| Comparative Example 6 | 28.1 | ○ | 150.0 |

(continued)

|  | skin permeability test permeation amount ($\mu$g/cm$^2$/24hr) | cohesion strength test | underwater constant-load peeling test peel rate (mm/min) |
|---|---|---|---|
| Comparative Example 7 | 45.6 | ○ | 230.8 |
| Comparative Example 8 | 40.8 | × | N.A. |
| Comparative Example 9 | 34.0 | × | N.A. |
| Comparative Example 10 | 63.2 | × | N.A. |
| Comparative Example 11 | 59.8 | × | N.A. |
| Comparative Example 12 | 62.0 | × | N.A. |
| Example 7 | 69.3 | ○ | 75.0 |
| Example 8 | 70.8 | ○ | 111.1 |
| Comparative Example 13 | 28.1 | ○ | 150.0 |
| Comparative Example 14 | 53.9 | ○ | 176.5 |
| Comparative Example 15 | 40.8 | × | N.A. |
| Comparative Example 16 | 52.9 | × | N.A. |
| Comparative Example 17 | 60.3 | × | N.A. |
| N.A.: not evaluated because threading was observed in cohesion strength test | | | |

[Table 7]

|  | skin permeability test permeation amount ($\mu$g/cm$^2$/24hr) | cohesion strength test | underwater constant-load peeling test peel rate (mm/min) |
|---|---|---|---|
| Example 9 | 164.2 | ○ | 107.1 |
| Example 10 | 153.7 | ○ | 130.4 |
| Example 11 | 170.9 | ○ | 136.4 |
| Comparative Example 18 | 153.3 | ○ | 200.0 |
| Comparative Example 19 | 130.5 | ○ | 200.0 |
| Comparative Example 20 | 147.4 | × | N.A. |
| Comparative Example 21 | 155.5 | × | N.A. |

(continued)

|  | skin permeability test permeation amount ($\mu$g/cm$^2$/ 24hr) | cohesion strength test | underwater constant-load peeling test peel rate (mm/min) |
|---|---|---|---|
| Comparative Example 22 | 161.4 | × | N.A. |
| Comparative Example 23 | 150.5 | × | N.A. |
| Example 12 | 174.9 | ○ | 100.0 |
| Example 13 | 157.0 | ○ | 136.4 |
| Example 14 | 156.9 | ○ | 176.5 |
| Comparative Example 24 | 153.3 | ○ | 200.0 |
| Comparative Example 25 | 128.1 | ○ | 250.0 |
| Comparative Example 26 | 147.4 | × | N.A. |
| Comparative Example 27 | 175.6 | × | N.A. |
| Comparative Example 28 | 143.3 | × | N.A. |
| Comparative Example 29 | 149.4 | × | N.A. |
| N.A.: not evaluated because threading was observed in cohesion strength test | | | |

The patch preparations containing levulinic acid and PGML (Examples 3 to 6 and Examples 9 to 11) showed high skin permeation amount and good adhesive properties (cohesion strength, water-resistant adhesiveness) compared with the patch preparations containing levulinic acid but not containing PGML (Comparative Example 6, Comparative Example 18), and the patch preparations containing PGML but not containing levulinic acid (Comparative Example 7, Comparative Example 19).

Similarly, the patch preparations containing levulinic acid and PGMC (Examples 7 to 8, Examples 12 to 14) showed high skin permeation amount and good adhesive properties (cohesion strength, water-resistant adhesiveness) compared with the patch preparations containing levulinic acid but not containing PGMC (Comparative Example 13, Comparative Example 24), and the patch preparations containing PGMC but not containing levulinic acid (Comparative Example 14, Comparative Example 25).

The patch preparations using lactic acid instead of levulinic acid (Comparative Examples 8 to 12, Comparative Examples 15 to 17, Comparative Examples 20 to 23, Comparative Examples 26 to 29) showed a threading phenomenon in the cohesion strength test, and cannot be used from the aspect of adhesive properties.

[Industrial Applicability]

According to the present invention, a patch preparation having good adhesive properties (cohesion strength, water-resistant adhesiveness) and superior in the skin permeability of drugs can be provided by containing a drug, levulinic acid, and propylene glycol fatty acid ester in the adhesive layer.

This application is based on a patent application No. 2020-026339 filed in Japan (filing date: February 19, 2020), the contents of which are incorporated in full herein.

**Claims**

1.    A patch preparation comprising a support and an adhesive layer on at least one surface of the support,

wherein the adhesive layer comprises a drug, levulinic acid,
and propylene glycol fatty acid ester, provided that the drug excludes tandospirone and a pharmaceutically acceptable salt thereof.

2. The patch preparation according to claim 1, wherein the aforementioned propylene glycol fatty acid ester is an ester of propylene glycol and saturated or unsaturated fatty acid having a carbon number of 8 to 18.

3. The patch preparation according to claim 1 or 2, wherein the aforementioned propylene glycol fatty acid ester is propylene glycol monocaprylate or propylene glycol monolaurate.

4. The patch preparation according to any one of claims 1 to 3, wherein the aforementioned adhesive layer further comprises other fatty acid ester other than the propylene glycol fatty acid ester.

5. The patch preparation according to claim 4, wherein the aforementioned other fatty acid ester is at least one kind selected from the group consisting of an ester of alcohol having a linear or branched chain alkyl group or cyclic alkyl group having a carbon number of 1 to 18 and saturated or unsaturated fatty acid having a carbon number of 8 to 18, and an ester of glycerol and saturated or unsaturated fatty acid having a carbon number of 8 to 18.

6. The patch preparation according to any one of claims 1 to 5, wherein a content of the aforementioned propylene glycol fatty acid ester is 1 to 30 mass % in 100 mass % of the adhesive layer.

7. The patch preparation according to any one of claims 1 to 6, wherein a content ratio of the levulinic acid and the propylene glycol fatty acid ester is 1:0.1 to 1:20 in mass ratio.

8. The patch preparation according to any one of claims 1 to 7, wherein an adhesive base constituting the aforementioned adhesive layer is an acrylic polymer.

9. The patch preparation according to any one of claims 1 to 8, wherein the aforementioned drug is a basic drug having a basic group.

10. The patch preparation according to any one of claims 1 to 9, wherein the aforementioned drug has a logPow of -1 to 7.

[Fig. 1]

(a)

(b)

## INTERNATIONAL SEARCH REPORT

| International application No. |
|---|
| PCT/JP2021/006051 |

**A. CLASSIFICATION OF SUBJECT MATTER**
A61K 9/70(2006.01)i; A61K 47/12(2006.01)i; A61K 47/14(2006.01)i; A61K 47/32(2006.01)i; A61K 31/137(2006.01)i
FI: A61K9/70 401; A61K47/12; A61K47/14; A61K31/137; A61K47/32
According to International Patent Classification (IPC) or to both national classification and IPC

**B. FIELDS SEARCHED**

Minimum documentation searched (classification system followed by classification symbols)
A61K9/70; A61K47/12; A61K47/14; A61K47/32; A61K31/137

Documentation searched other than minimum documentation to the extent that such documents are included in the fields searched
Published examined utility model applications of Japan        1922–1996
Published unexamined utility model applications of Japan      1971–2021
Registered utility model specifications of Japan              1996–2021
Published registered utility model applications of Japan      1994–2021

Electronic data base consulted during the international search (name of data base and, where practicable, search terms used)
JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2018-518502 A (CORIUM INTERNATIONAL, INC.) 12 July 2018 (2018-07-12) claims, examples, etc. | 1-8 |
| A | WO 2006/082728 A1 (HISAMITSU PHARMACEUTICAL CO., INC.) 10 August 2006 (2006-08-10) paragraph [0017] | 1-8 |

☐ Further documents are listed in the continuation of Box C.        ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 06 April 2021 (06.04.2021) | 13 April 2021 (13.04.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| Patent Documents referred in the Report | Publication Date | Patent Family | Publication Date |
|---|---|---|---|
| JP 2018-518502 A | 12 Jul. 2018 | WO 2016/209982 A1 claims, examples US 2018/0185298 A1 EP 3310348 A1 CN 108135860 A | |
| WO 2006/082728 A1 | 10 Aug. 2006 | US 2008/0138388 A1 paragraph [0028] | |

Form PCT/ISA/210 (patent family annex) (January 2015)

**INTERNATIONAL SEARCH REPORT**
Information on patent family members

International application No.

PCT/JP2021/006051

## REFERENCES CITED IN THE DESCRIPTION

**Patent documents cited in the description**

- JP 10251145 A
- JP 7247221 A
- JP 2020026339 A

**Non-patent literature cited in the description**

- *OECD GUIDELINE FOR THE TESTING OF CHEM-ICALS,* 27 July 1995, vol. 107